Europäisches Patentamt

(19) European Patent Office

Office européen des brevets

(11) Publication number: **0 292 080 B1**

## (12) EUROPEAN PATENT SPECIFICATION

(45) Date of publication of patent specification :
16.10.91 Bulletin 91/42

(51) Int. Cl.$^5$: **A61F 13/02**

(21) Application number: **88201036.6**

(22) Date of filing: **20.05.88**

(54) Weldable microporous non-woven tape for medical applications.

(30) Priority: **22.05.87 NL 8701228**

(43) Date of publication of application:
**23.11.88 Bulletin 88/47**

(45) Publication of the grant of the patent:
**16.10.91 Bulletin 91/42**

(84) Designated Contracting States:
**BE DE ES FR GB IT NL SE**

(56) References cited:
EP-A- 0 081 987
EP-A- 0 257 133
GB-A- 448 164
US-A- 3 645 835

(73) Proprietor: **AVERY DENNISON CORPORATION**
**150 North Orange Grove Boulevard**
**Pasadena California 91103 (US)**

(72) Inventor: **van Soom, Petrus Ludovicus Augustinus Maria**
**Dopheistraat 4**
**B-2350 Vosselaar (BE)**
Inventor: **van Hooijdonk, Adrianus Cornelis Petronella**
**Steenweg op Oosthoven 27 b5**
**B-2300 TURNHOUT (BE)**

(74) Representative: **Kooy, Leendert Willem et al**
**OCTROOIBUREAU VRIESENDORP & GAADE**
**P.O. Box 266**
**NL-2501 AW The Hague (NL)**

## Description

The present invention relates to a microporous laminated product to be adhered to human skin by means of an adhesive layer harmless to the skin, providing the unique combination of both moisture-vapour permeability and hydrostatic-pressure resistance that is to say it provides a liquid barrier. Said product may also be welded to a thermoplastic film. This product is highly suitable to be used as self-adhesive flange for stoma bags. Beside the use as a self-adhesive welding flange for stoma bags, this product is also suitable for the use as surgical plaster due to its vapour permeability, so that no moist film will occur between the adhesive layer and the human skin which would result in a loss of adhesive qualities and an uncomfortable feeling for the person, and in a loss of the liquid barrier, so the mechanical qualities of the product are maintained and a bacterial barrier is produced, so that the risk of exogeneous bacterial infection to such a plaster-covered wound is minimized.

Another application is the use of said product adhered to human skin, including the optimal use of thermoplastic auxiliaries fused upon the non-adhered side such as for example an electrocardiogram electrode.

The numerous kinds of commercially available stoma bags for both colostomia- and ileostomia patients consist of a self-adhesive flange which has as a function that the stoma bag can be attached to the stoma with a self-adhesive tape construction. Self-adhesive products are also commercially available, said products show a certain MVTR (= moisture-vapour transmission rate), to allow constant breathing of the skin when covered with tape. However, this implies that an open construction is to be made which has as a drawback that this is hardly or not moisture proof, in other words water from the outside contacting the tape, for example when the stoma patient with the stoma bag adhered to the skin would take a shower or bath.

An internal market study of a number of European ostomia bags' manufacturers showed that there is a demand for self-adhesive products meeting the following requirements :

— non-irritating pressure-sensitive adhesive
— moisture vapour permeable web
— moisture repellent non-adhered side
— non-adhered side fusable by means of heat, and/or high frequency radio waves to a thermoplastic film
— strong i.e. hard to tear, but flexible web.

Due to the fact that the thermoplastic film can be welded to the non adhered side of the web, the stoma-bags' manufacturer is in a position to produce the stoma bag in a more efficient and economical way.

Microporosity and moisture vapour permeability can be measured in various ways and a possibility is to measure the amount of air expressed in ml/min by a known surface at a certain pressure. However, the most relevant and practice-based test, is measurement of the MVTR (moisture vapour permeability rate) expressed in g $H_2O/m^2/24$ h at 37°C. The water-repellency may be measured by means of water retention in the web after its immersion. Another method to measure the water repellency is to measure the time up to leakage caused by a static liquid column of a specified height positioned on the product.

GB-A-448164 discloses a laminated product comprising a porous web of natural fibres (gauze) (3), to which an adhesive coating layer is applied. The gauze (3) is impregnated with an ester of acrylic acid, which is generally a thermoplastic material. The ester of acrylic acid is intended to act as a surfactant i.e. it has the effect of altering the interfacial tension of water.

Also US-A-3645835 discloses a laminated product designed to achieve the same object i.e. a laminated product having a high moisture vapour transition rate and being non-permeable to liquid water. The laminated product comprises a nonwoven web of synthetic fibres, to which an adhesive coating layer is applied. Although the product is not actually coated or impregnated with a thermoplastic material and surfactant, the non-woven layer of synthetic fibres comprises a copolymer of ethoxy ethyl methacrylate and methoxy ethyl methacrylate which is a thermoplastic and surfactant.

The singular feature of the invention is the balancing of the moisture vapour permeability and the liquid barrier which seems to be contradictory.

A microporous construction will show a high MVTR, but otherwise will have quite a low water barrier. A high water barrier can be achieved by sealing the web completely by means of a film, but then a very low MVTR will be achieved.

By coating and/or impregnating the web with a water-repellent agent and subsequently coating the web with a microporous adhesive, this unique combination is achieved. In case the MVTR is plotted as a function of the liquid barrier a line is obtained which dependent on the microporosity of the adhesive layer, the degree of water repellency which can be obtained via a plurality of techniques, and the density of tie nonwoven, will shift to the right to a limit as described herebelow. By an appropriate selection of the applied materials it is also possible to prepare the non-adhered side of the non-woven for welding to a thermoplastic film.

The invention relates to a microporous lamited product comprising a porous woven or non-woven web of paper, synthetic or natural fibers, said web being coated and/or impregnated with thermoplastic material, an adhesive coating layer, and optionally a protective release liner, characterized in that the web is also coated and/or impregnated with a surfactant, this surfactant being a fluorocarbon. Said product can have a moisture vapour transmission rate independent of the direction of at least 150 g/m²/24 h at 37°C and at the same time it can withstand a hydrostatic column of a 0.9% NaCl/H₂O solution of 11.5 cm height for at least 20 seconds without leakage.

A preferred product is has either a liquid barrier between 20 and 350 seconds measured with a 11.5 cm static liquid column of a 0.9 NaCl/H₂O solution and a moisture-vapour permeability between 150 and 3000 g/m²/24 h at 37°C or a liquid barrier between 350 and 1000 seconds measured with a 11.5 cm static liquid column of a 0.9% NaCl/H₂O solution and a moisture permeability between 150 and 1400 g/m²/24 h at 37° C.

Preferably the non-adhered side of the product is weldable to a synthetic film by means of heat, high frequency radio waves or ultrasonic techniques.

The thermoplastic material is preferably polyvinyl acetate.

## Examples

The present invention can be achieved in various ways discussed here below in detail. A final selection of production depends on the various technical possibilities.

1) A first possibility comprises the impregnation, spraying or foam-coating of a non-woven with an emulsion consisting of a fluorocarbon providing the water-repellency of the total construction and a polyvinyl chloride or polyvinyl acetate to make it possible in the final application to weld thermally by means of heat or high frequency radio waves a thermoplastic copolymer film of ethylvinyl acetate/polyethylene or a coextruded film consisting of polyvinylidene chloride/polyethylene to the treated non-woven. In a second step a microporous pressure-sensitive adhesive layer harmless to the skin is attached to the non-woven by means of a direct or transfer coating.

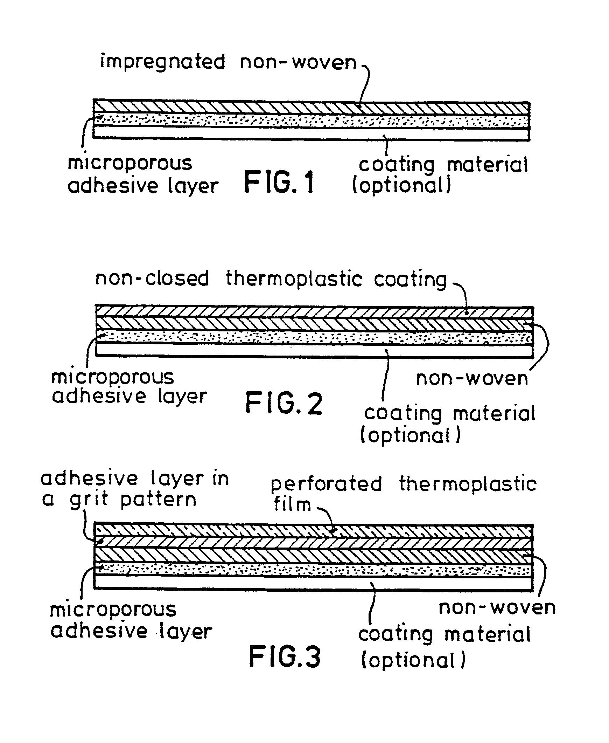

Producing a microporous adhesive can be done chemically by starting a chemical reaction for gas-producing after applying the adhesive layer to the support. A physical way is mixing air or an inert gas in the adhesive prior to or during the coating of the support with the adhesive layer. In both cases micropores are produced in the adhesive layer to provide the porous character. The whole construction may optionally be coated with siliconized coating material dependent on the final use and/or used coating technique (vide fig. 1).

MVTR values of the material may vary of about 1000 to 3000 g/m²/24 h/ at 37° C and a water repellency measured with a 11.5 cm high liquid column of a 0.9% NaCl/H₂O solution of some seconds to minutes dependent on the thickness of the adhesive layer, the absolute adhesive area i.e. microporosity percentage and the density of the non-woven. A typical example is the impregnation of a 35 g/m² 100% polyamide spunlaid non-woven with an emulsion consisting of a fluorocarbon and polyvinyl acetate.

Thereto a 30 g/m² microporous polyacrylate adhesive is applied by means of a transfer coating technique. The tape construction shows a MVTR of 2900 g/m²/24 h at 37°C and a water repellency of 180 sec.

2) A second possibility is the coating with a roller- or extrusion coating of a thermoplastic material, in this case ethylvinyl acetate on a non-woven. However, this has to provide a non-closed film to be obtained by a correct selection of the applied amount and the coating temperature, in order to obtain penetration of the coating into the non-woven, to produce a non-closed film. Subsequently a microporous layer can be applied to the non-coated side of the non-woven as described above sub 1). The water-repellency can be enhanced by treating the non-woven in a preceding step with a surfactant, e.g. fluorocarbon (vide fig. 2).

An example of said method is applying a 25 g/m² hot melt EVA by means of a die-coating to a water-repellent 35 g/m² 100% polyamide spunlaid non-woven, thereafter applying a 30 g/m² microporous polyacrylate adhesive to the non-EVA coated side with a transfer-coating technique. By means of this technique identical values can be obtained as described sub 1). Here it is also possible dependent on the final application to change the MVTR and/or water barrier dependent on the selection of the thickness of the adhesive layer, the absolute adhesive area i.e. microporosity, the density of the non-woven, the water-repellency of the non-woven, and the thickness/sealing of the EVA coating layer.

3) A third possibility is the application of a perforated thermoplastic film to the non-woven. This application may take place by means of heat, and/or pressure-lamination or by adhering. However, the adhesive medium should be a non-closed layer, because otherwise the result is a too closed construction with too low a MVTR. Therefore it is recommendable to apply the adhesive layer in a grit pattern.

In a further step a microporous adhesive layer is also applied as described sub 1). Optionally the liquid barrier may further be enhanced by using a water-repellent non-woven (vide fig. 3).

An example of the above described manner is heat laminating of a 25 microns 60 mesh/100 microns average perforated polyethylene film on a water-repellent 35 g/m² 100% polyamide spunlaid non-woven.

In a further step a 30 g/m² microporous polyacrylate adhesive layer is applied to the non-laminated side of the non-woven. Characterizing values are a MVTR of 2900 g/m²/24 h at 37°C and a water-repellency of 180 sec.

An alternative method is heat lamination and/or pressure lamination, or by extrusion of a sealed thermoplastic film on the non-woven, and in a further step this laminate is physically microperforated with e.g. a needle roller, laser beam, or a highpressure water jet. Subsequently a microporous adhesive layer optionally covered with a protective release liner is applied to said treated laminate.

## Claims

1. A microporous laminated product comprising a porous woven or non-woven web of paper, synthetic or natural fibers, said web being coated and/or impregnated with thermoplastic material, an adhesive coating layer, and optional a protective release liner, characterized in that the web is also coated and/or impregnated with a surfactant, this surfactant being a fluorocarbon.

## Patentansprüche

1. Mikroporöses laminiertes Produkt, umfassend eine poröse gewobene oder nichtgewobene Bahn aus Papier, Kunst- oder Naturfasern, wobei diese Bahn mit thermoplastischem Material, einer Klebstoff-Deckschicht, und wahlweise einer schützenden Trennlage beschichtet und/oder imprägniert ist, **dadurch gekennzeichnet,** daß die Bahn außerdem mit einem oberflächenaktiven Stoff beschichtet und-/oder imprägniert ist und dieser oberflächenaktive Stoff ein Fluorkohlenwasserstoff ist.

## Revendications

1. Produit laminé microporeux comportant une bande de papier poreux tissé ou non-tissé, des fibres synthétiques ou naturelles, ladite bande étant recouverte et/ou imprégnée d'une matière thermoplastique, une couche de revêtement adhésif, une couche optionnelle protectrice de séparation, caractérisé en ce que la bande est également revêtue et/ou imprégnée d'un agent de surface, cet agent de surface étant un fluorocarbone.

impregnated non-woven

microporous
adhesive layer          FIG. 1          coating material
                                        (optional)

non-closed thermoplastic coating

microporous
adhesive layer          FIG. 2          non-woven
                                        coating material
                                        (optional)

adhesive layer in          perforated thermoplastic
a grit pattern             film

microporous
adhesive layer          non-woven
                        coating material
        FIG. 3          (optional)